Europäisches Patentamt
European Patent Office
Office européen des brevets

(11) Veröffentlichungsnummer: **0 290 958 A2**

(12) **EUROPÄISCHE PATENTANMELDUNG**

(21) Anmeldenummer: 88107222.7

(22) Anmeldetag: 05.05.88

(51) Int. Cl.⁴: **C07D 295/08 , C07D 211/18 , C07D 211/58 , C07D 243/08 , A61K 31/445 , A61K 31/495 , A61K 31/55**

Patentansprüche für folgende Vertragsstaaten: ES + GR.

(30) Priorität: **12.05.87 DE 3715763**

(43) Veröffentlichungstag der Anmeldung: **17.11.88 Patentblatt 88/46**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Rüger, Wolfgang, Dr.**
**Parkstrasse 10**
**D-6233 Kelkheim (Taunus)(DE)**
Erfinder: **Urbach, Hansjörg, Dr.**
**Le Lavandoustrasse 41**
**D-6242 Kronberg/Taunus(DE)**
Erfinder: **Kaiser, Joachim, Dr.**
**Fichtestrasse 12**
**D-6000 Frankfurt am Main(DE)**

(54) **Diarylalkyl-substituierte Alkylamine, Verfahren zu ihrer Herstellung, ihre Verwendung sowie sie enthaltende Arzneimittel.**

(57) Verbindungen I werden beschrieben

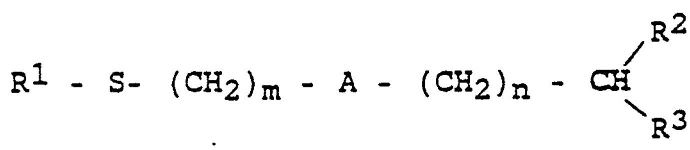

(I),

mit $R^1$ gleich (Cyclo-)Alk(en)yl oder

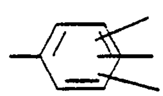

,

$R^2/R^3$ gleich Phenyl oder Phenylalkyl
A gleich

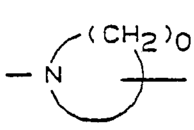

,

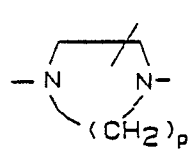

,

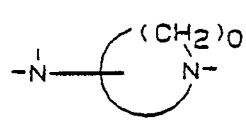

EP 0 290 958 A2

m gleich 2-4 und
n gleich 1-4.
Verbindungen I und ihre Salze sind Calciumantagonisten.

# Diarylalkyl-substituierte Alkylamine, Verfahren zu ihrer Herstellung, ihre Verwendung sowie sie enthaltende Arzneimittel

Die Erfindung betrifft Diarylalkyl-substituierte Alkylamine, Verfahren zu ihrer Herstellung, ihre Verwendung als Heilmittel und sie enthaltende Arzneimittel.

Zahlreiche Diarylbutylpiperidin-und Diarylbutylpiperazinderivate werden aufgrund ihrer Wirkung als Antagonisten des Dopamins therapeutisch als Neuroleptika genutzt. Strukturell verwandte Verbindungen aus der Reihe der Benzhydrylpiperazin-und der Diarylbutylpiperazinderivate wirken als Hemmstoffe des Einstroms von Calcium-Ionen in Zellen, wie z.B. Cinnarizin, Flunarizin und Lidoflazin. Sie finden Anwendung als Therapeutika zur Behandlung von Herz-Kreislauf-und cerebrovaskulären Erkrankungen.

N-Arylpiperazinalkanamide mit Diarylbutylsubstituenten an Piperazinsystemen werden in der Europäischen Patentanmeldung 68544 beschrieben: sie verbessern die Durchblutung des Herzens und schützen es vor den Folgen einer Ischämie, Anoxie oder Hypoxie.

Die spanische Patentschrift 504202 beschreibt Benzhydrylpiperazin-Derivate, in denen aber stets die Benzhydrylgruppe unmittelbar an den einen Stickstoff des Piperazins gebunden ist, nicht jedoch über eine $(CH_2)_n$-Brücke. Darüber hinaus zeigen diese Verbindungen lediglich eine vasodilatorische Wirkung, nicht aber eine calciumantagonistische Wirkung.

Es war daher überraschend, daß die Verbindungen der vorliegenden Erfindung den Einstrom von Calcium-Ionen in Zellen in ungewöhnlich starkem Ausmaß behindern. Sie eignen sich daher als Therapeutika zur Behandlung verschiedener Krankheiten, insbesondere des Herz-Kreislauf-Systems.

Die vorliegende Erfindung ist gerichtet auf Verbindungen der Formel I

$$R^1 - S - (CH_2)_m - A - (CH_2)_n - CH\begin{matrix} R^2 \\ R^3 \end{matrix} \quad (I),$$

die hervorragende calciumantagonistische Wirkung aufweisen und in welcher bedeuten:

$R^1$ $(C_1\text{-}C_6)$-Alkyl, geradkettig oder verzweigt, $(C_3\text{-}C_8)$-Cycloalkyl, $(C_2\text{-}C_6)$-Alkenyl, geradkettig oder verzweigt, $(C_5\text{-}C_8)$-Cycloalkenyl,

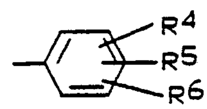

,

worin

$R^4$, $R^5$ und $R^6$ gleich oder verschieden unabhängig voneinander Wasserstoff, $(C_1\text{-}C_6)$-Alkyl, $(C_3\text{-}C_8)$-Cycloalkyl, Hydroxy, $(C_1\text{-}C_4)$-Alkoxy, $(C_1\text{-}C_4)$-Alkylthio, F, Cl, Br, J, Nitro, Cyano, Trifluormethyl, Formyl, Carboxyl, $(C_1\text{-}C_6)$-Alkoxycarbonyl, $(C_1\text{-}C_6)$-Acyl, Carbamoyl, N-Mono-oder N,N-Di-$(C_1\text{-}C_6)$-Alkylcarbamoyl, Sulfo, $(C_1\text{-}C_6)$-Alkoxysulfonyl, Sulfamoyl, N-Mono-oder N,N-Di-$(C_1\text{-}C_6)$-Alkylsulfamoyl, $(C_1\text{-}C_6)$-Alkylsulfinyl, $(C_1\text{-}C_6)$-Alkylsulfonyl, Amino, unsubstituiert oder substituiert mit ein oder zwei gleich-oder verschiedenartigen $(C_1\text{-}C_6)$-Alkyl-, $(C_1\text{-}C_6)$-Acyl-oder Aryl-, vorzugsweise Phenylgruppen,

$R^2$ und $R^3$ gleich oder verschieden und unabhängig voneinander Phenyl, Phenyl-$(C_1\text{-}C_4)$-alkyl, wobei der Phenylring jeweils unsubstituiert oder durch einen, zwei oder drei Substituenten aus der Gruppe $(C_1\text{-}C_4)$-Alkyl, $(C_1\text{-}C_4)$-Alkoxy, F, Cl, Br, J, Cyano, Nitro oder Trifluormethyl substituiert ist,

A ein Amin

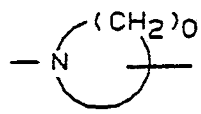 , 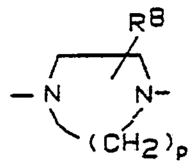 oder 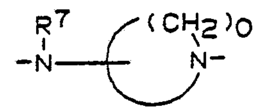

worin

$R^7$ Wasserstoff, ($C_1$-$C_6$)-Alkyl, Aryl, vorzugsweise Phenyl,
$R^8$ Wasserstoff, ($C_1$-$C_6$)-Alkyl, Formyl, ($C_1$-$C_6$)-Acyl, Carboxyl, ($C_1$-$C_6$)-Alkoxycarbonyl, Carbamoyl, N-Mono-oder N,N-Di-($C_1$-$C_6$)-Alkylcarbamoyl,
o 3, 4, 5, 6 oder 7,
p 2 oder 3, bedeuten,
m 2, 3 oder 4
n 1, 2, 3 oder 4,

sowie die Salze der Verbindungen der Formel I mit physiologisch verträglichen Säuren.

Bevorzugt werden Verbindungen der Formel I, in welcher mindestens einer der Reste und Indices folgende Bedeutung hat:
$R^1$ ($C_3$-$C_8$)-Cycloalkyl,

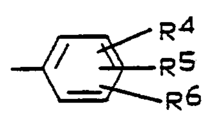 ,

worin

$R^4$, $R^5$ und $R^6$ gleich oder verschieden unabhängig voneinander Wasserstoff, ($C_1$-$C_6$)-Alkyl, ($C_1$-$C_4$)-Alkoxy, ($C_1$-$C_4$)-Alkylthio, F, Cl, Br, J, Nitro, Cyano, Trifluormethyl, Formyl, Carboxyl, ($C_1$-$C_6$)-Alkoxycarbonyl, ($C_1$-$C_6$)-Acyl, Carbamoyl, N-Mono-oder N,N-Di-($C_1$-$C_6$)-Alkylcarbamoyl, Sulfo, Sulfamoyl, N-Mono-oder N,N-Di-($C_1$-$C_6$)-Alkylsulfamoyl, ($C_1$-$C_6$)-Alkylsulfinyl, ($C_1$-$C_6$)-Alkylsulfonyl bedeuten,

$R^2$ und $R^3$ gleich oder verschieden und unabhängig voneinander Phenyl, Phenyl-methyl, wobei der Phenylring jeweils unsubstituiert oder durch einen, zwei oder drei Substituenten aus der Gruppe Methyl, Ethyl, Methoxy, Ethoxy, F, Cl, Br, J, Cyano, Nitro oder Trifluormethyl substituiert ist,
A ein Amin

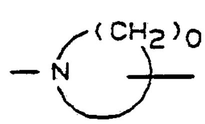 , 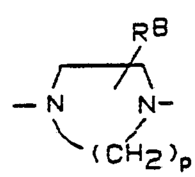 oder 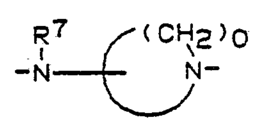

worin

$R^7$ Wasserstoff, Methyl, Ethyl,
$R^8$ Wasserstoff, Carboxyl, Carbamoyl,
o 4, 5 oder 6,
p 2 oder 3 bedeuten,
m - 2, 3 oder 4

n 1, 2, 3 oder 4,

sowie die Salze der Verbindungen der Formel I mit physiologisch verträglichen Säuren.

Besonders bevorzugt werden Verbindungen der Formel I, in welcher mindestens einer der Substituenten und Indices folgende Bedeutung hat:

$R^1$ ($C_5$-$C_7$)-Cycloalkyl,

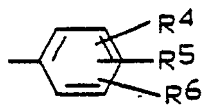

,

worin

$R^4$, $R^5$ und $R^6$ gleich oder verschieden unabhängig voneinander Wasserstoff, Methyl, Ethyl, Propyl, Isopropyl, n-Butyl, sec-Butyl, Isobutyl, tert. Butyl, Methoxy, Ethoxy, Methylthio, Fluor, Chlor, Brom, Jod, Nitro, Cyano, Trifluormethyl, Acetyl, Methylsulfonyl, Methylsulfinyl,

$R^2$ und $R^3$ gleich oder verschieden unabhängig voneinader Phenyl, Phenyl-methyl, wobei der Phenylring jeweils unsubstituiert oder durch einen, zwei oder drei Substituenten aus der Gruppe Methyl, Fluor, Chlor, Brom, Cyano, Nitro oder Trifluormethyl substituiert ist,

A ein Amin

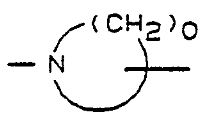

,

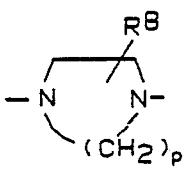

,

worin

$R^8$ Wasserstoff, Carboxyl, Carbamoyl,

o 4, 5 oder 6,

p 2 oder 3 bedeuten,

m 2, 3 oder 4

n 2, 3 oder 4,

sowie die Salze der Verbindungen der Formel I mit physiologisch verträglichen Säuren.

Ganz besonders bevorzugt werden Verbindungen der Formel I, in welcher mindestens einer der Substituenten oder Indices folgende Bedeutung hat:

$R^1$

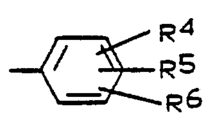

,

worin

$R^4$, $R^5$ und $R^6$ gleich oder verschieden unabhängig voneinander Wasserstoff, Methyl, tert.Butyl, Methoxy, Methylthio, Fluor, Chlor, Brom, Nitro, Cyano, Trifluormethyl, Acetyl, Methylsulfonyl, Methylsulfinyl bedeuten,

$R^2$ und $R^3$ gleich oder verschieden unabhängig voneinander Phenyl, Phenyl-methyl, wobei der Phenylring jeweils unsubstituiert oder durch Fluor oder Trifluormethyl substituiert ist,

A ein Amin

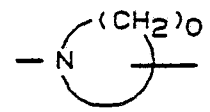, 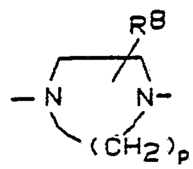,

worin

$R^8$ Wasserstoff,
o 5,
p 2 bedeuten,
m 2,
n 3,

sowie die Salze der Verbindungen der Formel I mit physiologisch verträglichen Säuren.

Als solche Säuren kommen beispielsweise anorganische Säuren wie Salzsäure, Bromwasserstoffsäure, Jodwasserstoffsäure, Schwefelsäure, Phosphorsäure oder Salpetersäure oder organische Säuren wie Weinsäure, Citronensäure, Äpfelsäure, Milchsäure, Maleinsäure, Fumarsäure, Malonsäure, Oxalsäure, Essigsäure, Propionsäure, Methansulfonsäure, Benzolsulfonsäure, p-Toluolsulfonsäure, Naphthalin-1,5-disulfonsäure oder Gluconsäure in Betracht.

Die Verbindungen der Formel I weisen zum Teil asymmetrische Kohlenstoffatome auf und können daher als Enantiomere und Diastereomere auftreten. Die Erfindung umfaßt sowohl die reinen Enantiomeren als auch die Racemate. Die Racemate können nach gebräuchlichen Methoden, zum Beispiel durch Salzbildung mit optisch aktiven Säuren wie Camphersulfonsäure oder Di-benzoylweinsäure, fraktionierte Kristallisation und anschließende Freisetzung der Basen aus ihren Salzen, oder durch Derivatisierung mit geeigneten optisch aktiven Reagenzien, Trennung der diastereomeren Derivate durch fraktionierte Kristallisation oder Chromatographie an Kieselgel oder Aluminiumoxid und Rückspaltung in die Enantiomeren aufgetrennt werden. Die Diastereomeren können nach gebräuchlichen Methoden, wie fraktionierte Kristallisation oder Chromatographie an Säulen, getrennt werden.

Die vorliegende Erfindung betrifft weiterhin ein Verfahren zur Herstellung von Verbindungen der Formel I, das dadurch gekennzeichnet ist, daß man

a) eine Verbindung der Formel II,

$R^1 - S - (CH_2)_m - Y$ (II),

in welcher $R^1$ und m die gleiche Bedeutung wie in Formel I haben, und in welcher Y eine Abgangsgruppe, die nucleophil verdrängt werden kann, insbesondere ein Cl, Br, J-Atom oder einen Sulfonsäurerest, vorzugsweise einen Methansulfonyl-, Benzolsulfonyl-, p-Toluolsulfonyl-oder Trifluormethansulfonylrest bedeutet, mit einer Verbindung der Formel III,

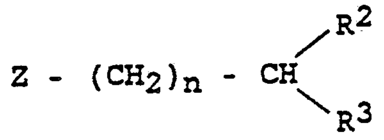 (III)

in welcher $R^2$, $R^3$ und n die gleiche Bedeutung wie in Formel I haben, und in welcher Z

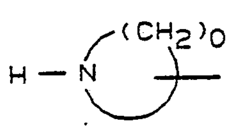, 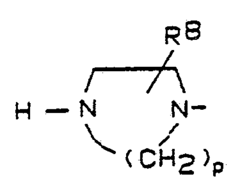 oder 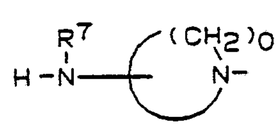

bedeutet, worin

$R^7$, $R^8$, o und p die gleiche Bedeutung wie in Formel I haben, unter Bedingungen einer nucleophilen Substitution, vorzugsweise in einem polaren organischen Lösungsmittel, wie einem Alkohol, vorzugsweise Methanol, Ethanol, Propanol oder Isopropanol oder einem niederen Keton, vorzugsweise Aceton, Methylethylketon oder Methylisobutylketon oder in Acetonitril, Dimethylformamid, Dimethylsulfoxid oder Sulfolan oder einem Kohlenwasserstoff, vorzugsweise Toluol, mit oder ohne Gegenwart einer Hilfsbase zum Abfangen der sich bildenden Säure, vorzugsweise in Gegenwart von Kaliumcarbonat, Natriumcarbonat, Triethylamin, Pyridin, 1,5-Diazabicyclo[5,4,0]undec-5-en oder 1,5-Diazabicyclo[4,3,0]non-5-en, sowie mit oder ohne Gegenwart eines Alkalihalogenids, vorzugsweise Natriumjodid oder Kaliumjodid, bei einer Temperatur zwischen 0 und 160 °C, vorzugsweise zwischen 20 und 120 °C, umsetzt, oder daß man

b) eine Verbindung der Formel IV,

$R^1 - S - (CH_2)_m - Z$ (IV),

in welcher $R^1$ und m die gleiche Bedeutung wie in Formel I und Z die gleiche Bedeutung wie in Formel III haben, mit einer Verbindung der Formel V,

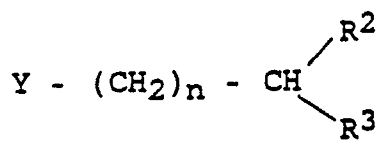 (V),

in welcher $R^2$, $R^3$ und n die gleiche Bedeutung wie in Formel I und Y die gleiche Bedeutung wie in Formel II haben, unter den Bedingungen einer nucleophilen Substitution, wie unter a) beschrieben, umsetzt, oder daß man

c) eine Verbindung der Formel VI,

$R^1 - SH$ (VI)

in der $R^1$ die gleiche Bedeutung wie in Formel I besitzt, mit einer Verbindung der Formel VII,

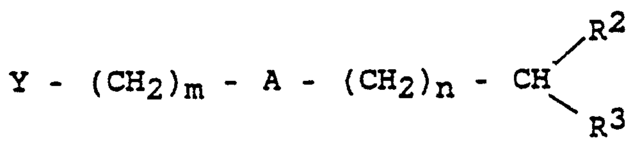 (VII),

in der $R^2$, $R^3$, A, m und n die gleiche Bedeutung wie in Formel I und Y die gleiche Bedeutung wie in Formel II haben, entweder in einem polaren aprotischen Lösungsmittel wie Acetonitril, Tetrahydrofuran, Dimethylsulfoxid, Dimethylformamid, Sulfolan oder N-Methylpyrrolidon, in Gegenwart einer starken Base, wie Natriumhydrid, Kaliumhydrid, Natriumamid, Lithiumdiisopropylamid, Butyllithium oder Lithiumhexamethyldisilazid, vorzugsweise in Dimethylformamid oder Dimethylsulfoxid in Gegenwart von Natriumhydrid oder Natriumamid, bei einer Temperatur zwischen -40 ° und +100 °C, vorzugsweise zwischen -20 ° und +50 °C, oder in einem protischen oder aprotischen polaren organischen Lösungsmittel, wie einem niederen Alkohol, beispielsweise Methanol, Ethanol, Isopropanol, oder einem niederen Keton, vorzugsweise Aceton, Methylethylketon oder Methylisobutylketon, oder in Dimethylformamid, in Gegenwart einer schwachen bis mittelstarken Base, wie einem Alkali-oder Erdalkalimethallhydroxid oder -carbonat, vorzugsweise Natriumcarbonat oder Kaliumcarbonat, oder einem Amin, wie beispielsweise Triethylamin, Pyridin, N-Ethyldiisopropylamin, 1,5-Diazabicyclo[5,4,0]undec-5-en oder 1,5-Diazabicyclo[4,3,0]non-5-en bei einer Temperatur zwischen 0 ° und 160 °C, vorzugsweise zwischen 20 ° und 120 °C, umsetzt oder daß man

d) eine Verbindung der Formel VIII,

$R^1 - W$ (VIII)

in der $R^1$ die gleiche Bedeutung wie in Formel I besitzt und in der W eine Abgangsgruppe, die nucleophil verdrängt werden kann, insbesondere ein Fluor-, Chlor-, Brom-oder Iodatom, eine Nitro-, Hydroxy-, Alkoxy- oder Trialkylammoniumgruppe oder einen Sulfonsäurerest, vorzugsweise ein Fluor-oder Chloratom, eine Nitrogruppe oder einen Methansulfonyl-, Benzolsulfonyl-, p-Toluolsulfonyl-oder Trifluormethansulfonylrest bedeutet, mit einer Verbindung der Formel IX,

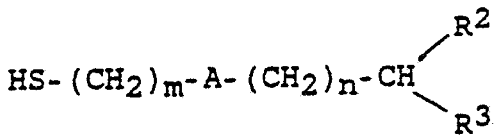 (IX)

in der $R^2$, $R^3$, A, m und n die gleiche Bedeutung wie in Formel I besitzen, unter Bedingungen einer nucleophilen Substitution, beispielsweise ohne Lösungsmittel oder in einem wäßrigen Lösungsmittel, vorzugsweise in einem polaren organischen Lösungsmittel wie einem Alkohol, bevorzugt Methanol, Ethanol, Propanol oder Isopropanol, oder einem Keton, bevorzugt Aceton, Methylethylketon oder Methylisobutylketon, oder einem Ether, bevorzugt Diethylether, tert. Butylmethylether, Dimethoxyethan, Tetrahydrofuran oder Dioxan, oder einem halogenierten Kohlenwasserstoff, bevorzugt Dichlormethan, Chloroform oder 1,2-Dichlorethan, oder in Acetonitril, Dimethylformamid, Dimethylsulfoxid oder Sulfolan, oder in einem Kohlenwasserstoff, bevorzugt Benzol oder Toluol, oder in einem gemischten wäßrig-organischen Lösungsmittelsystem unter Zusatz eines Phasentransferkatalysators, mit oder ohne Gegenwart einer Hilfsbase zum Abfangen der sich bildenden Säure, vorzugsweise in Gegenwart von Kaliumcarbonat, Natriumcarbonat, Triethylamin, Pyridin, Lithiumdiisopropylamid, n-Butyllithium, Natriumhydrid, Kaliumhydrid, Natriumamid, 1,5-Diazabicyclo-[5.4.0]undec-5-en oder 1,5-Diazabicyclo[4.3.0]-non-5-en, sowie mit oder ohne Gegenwart von Kupferpulver bei einer Temperatur zwischen -80° und +200° C, vorzugsweise zwischen -30° und +120° C, umsetzt.

Die Verbindungen der Formel II sind literaturbekannt bzw. lassen sich unter analogen Bedingungen aus Verbindungen der Formel VI durch Umsetzung mit α,ω-Dihalogenalkanen oder ω-Halogenalkylsulfonaten herstellen.

Die Herstellung der Verbindungen der Formel III ist in der deutschen Patentanmeldung P 36 00 390.5 wie folgt vorgeschlagen worden:

Die Verbindungen der Formel III, mit Z gleich

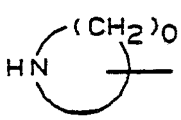 , 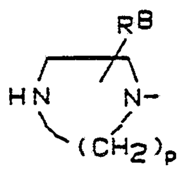 oder 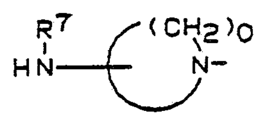

worin $R^7$, $R^8$, o und p die gleiche Bedeutung wie in Formel I haben, lassen sich in an sich bekannter Weise aus Verbindungen der Formel X

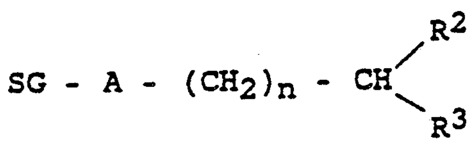 (X),

worin $R^2$, $R^3$, A und n die gleiche Bedeutung wie in Formel I haben und worin SG eine geeignete Schutzgruppe, wie z.B. eine Carbamat-, Amid-, Alkyl-oder Benzylgruppe, vorzugsweise eine Formyl-, Ethoxycarbonyl-, Benzyl-oder Tritylgruppe bedeutet, durch Abspaltung der Schutzgruppe unter literaturbekannten Bedingungen, z.B. saure oder alkalische Spaltung oder durch Hydrogenolyse, herstellen.

Die Verbindungen der Formel III, mit Z gleich

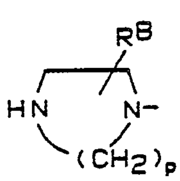 oder 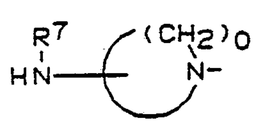 ,

worin $R^7$, $R^8$, o und p die gleiche Bedeutung wie in Formel I haben, lassen sich außerdem herstellen durch Umsetzung von Verbindungen der Formel V mit Aminen der Formel Z - H, wobei Z eine der obengenannten

Gruppen ist, unter den Bedingungen einer nucleophilen Substitution, wie sie unter der Verfahrensvariante a) beschrieben sind,

oder

durch Umsetzung von Verbindungen der Formel V mit geschützten Aminen

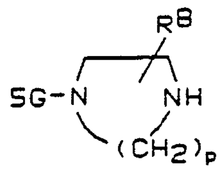

oder

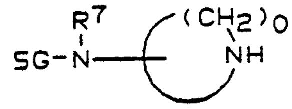

,

worin $R^7$, $R^8$, o und p die gleiche Bedeutung wie in Formel I und SG die gleiche Bedeutung wie in Formel X haben, unter den Bedingungen einer nucleophilen Substitution, wie sie unter der Verfahrensvariante a) beschrieben sind, mit anschließender Abspaltung der Schutzgruppe unter üblichen Bedingungen, z.B. durch saure oder alkalische Spaltung oder durch Hydrogenolyse.

Die Verbindungen der Formel III, in denen Z

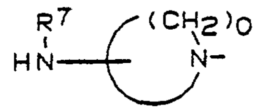

ist,

worin o die gleiche Bedeutung wie in Formel I hat und $R^7$ Methyl ist, lassen sich bevorzugt durch Reduktion mit geeigneten Reduktionsmitteln, vorzugsweise Lithiumaluminiumhydrid, aus Verbindungen der gleichen Formel, worin $R^7$ einen Alkoxycarbonylrest bedeutet, herstellen.

Die Verbindungen der Formel IV lassen sich aus den Verbindungen der Formel II nach analogen Verfahren, wie sie bereits für die Darstellung der Verbindungen der Formel III aus den Verbindungen der Formel V beschrieben sind, herstellen,

oder

aus Verbindungen der Formel XI

$R^1$ - S - $(CH_2)_m$ -A - SG (XI),

worin $R^1$, A und m die gleiche Bedeutung wie in Formel I und SG die gleiche Bedeutung wie in Formel X besitzen, durch Abspaltung der Schutzgruppe unter den üblichen Bedingungen, z.B. durch saure oder alkalische Spaltung oder durch Hydrogenolyse,

oder

aus Verbindungen der Formel VI durch Umsetzung mit Verbindungen der Formel XII

Y - $(CH_2)_m$ -A - SG (XII),

worin A und m die gleiche Bedeutung wie in Formel I, Y die gleiche Bedeutung wie in Formel II und SG die gleiche Bedeutung wie in Formel X besitzen, unter den Bedingungen einer Alkylierungsreaktion, wie sie unter der Verfahrensvariante c) beschrieben sind, gefolgt von einer Abspaltung der Schutzgruppe unter den üblichen Bedingungen.

Die Verbindungen der Formel V und VI sind großenteils literaturbekannt bzw. können in analoger Weise hergestellt werden.

Die Herstellung der Verbindungen der Formel VII ist in der deutschen Patentanmeldung P 36 00 390.5 wie folgt vorgeschlagen worden:

Die Verbindungen der Formel VII lassen sich herstellen aus den Verbindungen der Formel III durch Umsetzung mit α,ω-Dihalogenalkanen oder ω-Halogenalkylsulfonaten unter den Bedingungen einer nucleophilen Substitution, wie sie unter der Verfahrensvariante a) beschrieben sind

oder

aus den Verbindungen der Formel V durch Umsetzung mit einer Verbindung der Formel XIII

Y - $(CH_2)_m$ -Z (XIII)

worin m die gleiche Bedeutung wie in Formel I, Y die gleiche Bedeutung wie in Formel II und Z die gleiche Bedeutung wie in Formel III besitzen, unter den Bedingungen einer nucleophilen Substitution, wie sie unter der Verfahrensvariante a) beschrieben sind,

oder

aus den Verbindungen der Formel V durch Umsetzung mit einer Verbindung der Formel XIV
HO - $(CH_2)_m$ -Z (XIV),
worin m die gleiche Bedeutung wie in Formel I und Z die gleiche Bedeutung wie in Formel III besitzen, unter den Bedingungen einer nucleophilen Substitution, wie sie unter der Verfahrensvariante a) beschrieben sind, zu Verbindungen der Formel (XV) und anschließende Überführung der Hydroxyfunktion in die Abgangsgruppe Y nach gängigen Methoden.

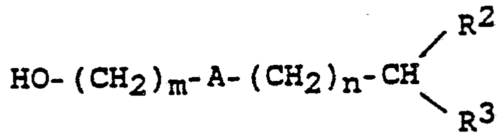
(XV),

Die Verbindungen der Formel VIII sind bekannt bzw. nach einfachen Verfahren aus käuflichen Ausgangsmaterialien zugänglich.

Die Verbindungen der Formel IX,

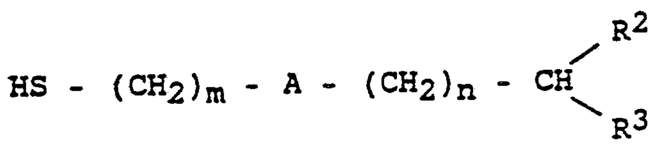
(IX),

worin $R^2$, $R^3$, A, m und n die gleiche Bedeutung wie in Formel I besitzen, lassen sich aus Verbindungen der Formel III durch Umsetzung mit Verbindungen der Formel XVI
HS - $(CH_2)_m$ -Y (XVI),
worin m die gleiche Bedeutung wie in Formel I und Y die gleiche Bedeutung wie in Formel II besitzt, unter den Bedingungen einer nucleophilen Substitution, wie sie unter der Verfahrensvariante a) beschrieben sind
oder
aus den Verbindungen der Formel V durch Umsetzung mit einer Verbindung der Formel XVII
HS - $(CH_2)_m$ -Z (XVII),
worin m die gleiche Bedeutung wie in Formel I und Z die gleiche Bedeutung wie in Formel III besitzen, unter den Bedingungen einer nucleophilen Substitution, wie sie unter der Verfahrensvariante a) beschrieben sind,
oder
aus den Verbindungen der Formel VII durch Substitution der Abgangsgruppe Y durch eine Mercaptogruppe nach einem der gängigen Verfahren herstellen.

Die Verbindungen der Formel X werden mit Hilfe der allgemeinen Verfahren der Schutzgruppenchemie aus den Verbindungen der Formel III hergestellt, oder aus den Verbindungen der Formel V durch Umsetzung mit geschützten Aminen, wie bereits bei der Herstellung von Verbindungen der Formel III aus solchen der Formel V beschrieben wurde.

Die Verbindungen der Formel XI werden mit Hilfe der allgemeinen Verfahren der Schutzgruppenchemie aus den Verbindungen der Formel IV hergestellt, oder aus den Verbindungen der Formel VI durch Umsetzung mit Verbindungen der Formel XII, wie bereits bei der Herstellung von Verbindungen der Formel IV aus solchen der Formel VI beschrieben wurde.

Die Verbindungen der Formel XII werden mit Hilfe der allgemeinen Verfahren der Schutzgruppenchemie aus den Verbindungen der Formel XIII hergestellt oder durch Umsetzung von $\alpha,\omega$-Dihalogenalkanen oder $\omega$-Halogenalkylsulfonaten mit geschützten Aminen, wie bereits bei der Herstellung von Verbindungen der Formel III aus solchen der Formel V beschrieben wurde.

Die Verbindungen der Formel XV lassen sich herstellen aus Verbindungen der Formel III durch Umsetzung mit Verbindungen der Formel XVIII
HO - $(CH_2)_m$ -Y (XVIII),
worin m die gleiche Bedeutung wie in Formel I und Y die gleiche Bedeutung wie in Formel II besitzt, unter den Bedingungen einer nucleophilen Substitution, wie sie unter der Verfahrensvariante a) beschrieben sind,
oder
aus den Verbindungen der Formel V durch Umsetzung mit einer Verbindung der Formel XIV, wie bei der Herstellung von Verbindungen der Formel VII aus solchen der Formel V beschrieben wurde.
Die Verbindungen der Formeln XIII, XIV, XVI, XVII und XVIII sind bekannt bzw. nach einfachen Verfahren

aus käuflichen Ausgangsmaterialien zugänglich.

Die erfindungsgemäßen Verbindungen der Formel I weisen biologische Wirkungen, insbesondere calciumantagonistische Wirkungen auf und besitzen daher wertvolle Eigenschaften zur Behandlung aller Krankheitszustände, die auf einer Störung im Calciumhaushalt beruhen: insbesondere sind sie als blutdrucksenkende Mittel, antianginös wirkende Mittel, antiarrhythmisch wirkende Mittel und zur Verbesserung der cerebralen Gefäßdurchblutung geeignet.

Ihre calciumantagonistische Wirksamkeit kann in dem biochemischen Testmodell der Verdrängung von tritiummarkierten Nitrendipin gezeigt werden. Diese Prüfung führten wir in einer aus dem Cortex des Rattenhirns gewonnenen und mehrfach gewaschenen Membranpräparation aus, wobei wir im wesentlichen die von R. J. Gould et al. (Proc. Natl. Acad. Sci. USA 79, 3656 [1982]) beschriebene Methode anwendeten. Die auf 1:1500 mit TRIS-Puffer pH 7,4 (50 mM TRIS-HCl, 150 mM NaCl, 1,0 mM $CaCl_2$ und 0,001 Gew.-%, bezogen auf TRIS-HCl, NaCl und $CaCl_2$ in Lösung, einer neutralen oberflächenaktiven Substanz, wie z.B. Genapol®) verdünnte Membransuspension wurde in 5 ml-Portionen mit $^3$H-Nitrendipin (0,1 nM im Test, spez. Aktivität 81,3 Ci/mMol) und mit den Prüfsubstanzen in verschiedenen Konzentrationen 60 Min. bei 25 °C im Schüttelwasserbad inkubiert. Die Abtrennung der Membranfraktionen wurde durch Vakuumfiltration über Whatman-GF/F-Glasfaserfilter vorgenommen und die Radioaktivität im Flüssigkeitsszintillationszähler gemessen. Die unspezifische $^3$H-Nitrendipin-Bindung bestimmten wir in Gegenwart von I µM Nifedipin. Als charakteristische Größe wird der $IC_{50}$-Wert bestimmt, d.h. diejenige Konzentration der Testsubstanz, die 50 % des radioaktiv markierten Nitrendipins zu verdrängen vermag.

In diesem Modell weisen die erfindungsgemäßen Verbindungen der Formel I $IC_{50}$-Werte von etwa $10^{-6}$ molar bis etwa $10^{-9}$ molar auf. Sie sind somit deutlich stärker wirksam als bekannte Vergleichsverbindungen wie Flunarizin und Lidoflazin.

Die folgende Tabelle enthält einige der gemessenen $IC_{50}$-Werte

| Beispiel Nr. | $IC_{50}$ ($10^{-9}$M) |
|---|---|
| 1 | 4 |
| 2 | 20 |
| 3 | 7 |
| 4 | 22 |
| 5 | 1,2 |
| Flunarizin | 1000 |
| Lidoflazin | 430 . |

In weiteren Testmodellen, mit denen calciumantagonistische Wirkung nachgewiesen werden kann, z.B. an der relaxierenden Wirkung auf das vorkontrahierte Meerschweinchenileum, am Aktionspotential des isolierten Meerschweinchenpapillarmuskels oder an der Wirkung auf die Coronardurchströmung des isolierten Meerschweinchenherzens, sind die Verbindungen der Formel I ebenfalls stark wirksam.

Die erfindungsgemäßen Verbindungen der Formel I und ihre pharmakologisch verträglichen Salze sind innerhalb eines breiten Dosisbereichs wirksam. Die Höhe der verabreichten Dosis ist abhängig von der Art der gewünschten Behandlung, von der Verabreichungsweise, vom Zustand, vom Typ und von der Größe des behandelten Patienten. Bei oraler Dosierung werden befriedigende Ergebnisse mit Dosen von ab 0,01 mg, vorzugsweise ab 0,1 mg und bis zu 100 mg, vorzugsweise bis zu 20 mg einer Verbindung der Formel I pro kg Körpergewicht erreicht. Beim Menschen variiert die tägliche Dosis zwischen 1 und 800 mg, vorzugsweise 2 bis 500 mg, wobei Einzeldosen von 0,5 bis 200 mg, vorzugsweise ein-bis dreimal täglich, gegeben werden können. Für intravenöse und intramuskuläre Anwendung beträgt die Dosis 0,1 bis 300 mg, vorzugsweise 0,5 bis 150 mg täglich.

Die pharmakologisch verwendbaren Verbindungen der vorliegenden Erfindung und ihre Salze können zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten oder Gelatinekapseln, welche den Wirkstoff zusammen mit Verdünnungsmitteln, z.B. Lactose, Dextrose, Rohrzucker, Mannitol, Sorbitol, Cellulose und/oder Glycin und Gleitmitteln wie Kieselerde, Talk, Stearinsäure oder deren Salze, wie Magnesium-oder

Calciumstearat, und/oder Polyethylenglykol enthalten. Tabletten enthalten ebenfalls Bindemittel wie Magnesiumaluminiumsilicat, Stärke, Gelatine, Traganath, Methylcellulose, Natriumcarboxymethylcellulose und/oder Polyvinylpyrrolidon und, falls benötigt, Farbstoffe, Geschmacksstoffe und Süßmittel. Injizierbare Lösungen sind vorzugsweise isotonische wäßrige Lösungen oder Suspensionen, die sterilisiert sein können und Hilfssstoffe wie Konservier-, Stabilisierungs-, Netz-und/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die, wenn erwünscht, weitere pharmakologisch wertvolle Stoffe enthalten können, werden z.B. mittels konventioneller Misch-, Granulier-, und Dragierverfahren, hergestellt und enthalten 0,1 % bis etwa 75 %, bevorzugt etwa 1 % bis etwa 50 % des Wirkstoffs.

Die im Anschluß folgenden Beispiele sollen die Erfindung erläutern, ohne sie auf diese Beispiele zu begrenzen.

**Beispiel 1.**

1-[4,4-Bis(4-fluorphenyl)butyl]-4-[2-(4-chlorphenylthio)-ethyl]piperazin

Zu einer Suspension von 0,35 g Natriumhydrid (55 % Dispersion in Öl) in 8 ml absoluten Dimethylformamid wurde eine Lösung von 1,15 g (8 mmol) 4-Chlorthiophenol in 20 ml absolutem Dimethylformamid langsam zugetropft und eine Stunde auf 40 °C geheizt. Nach Beendigung der Gasentwicklung wurde kurze Zeit auf 60 °C erwärmt, dann auf Zimmertemperatur abgekühlt und eine Lösung von 3,05 g (7,7 mmol) 1-[4,4-Bis(4-fluorphenyl)butyl]-4-(2-chlorethyl)piperazin in 25 ml absolutem Dimethylformamid zugegeben und 24 Stunden bei Zimmertemperatur gerührt. Die Lösung wurde auf 110 ml 0,2 N Natronlauge gegeben, mit Methylenchlorid extrahiert, der Extrakt über Magnesiumsulfat getrocknet, eingeengt und durch Säulenchromatographie an Kieselgel (Toluol/Ethanol 9 : 1) gereinigt. Ausbeute: 2,6 g (67 %) öliges Produkt. Durch Lösen dieses Öls in Essigester und Behandlung mit etherischer HCl wurde das kristalline Dihydrochlorid erhalten, Schmp. 244 °C (Zers.).

Auf einem analogen Herstellungswege wurden die folgenden Produkte erhalten.

**Beispiel 2:**

1-[4,4-Bis-(4-fluorphenyl)butyl]-4-[2-(4-methylphenylthio)ethyl]piperazin-dihydrochlorid, Schmp. 235-238 °C.

**Beispiel 3**

1-[4,4-Bis(4-fluorphenyl)butyl]-4-[2-(4-fluorphenylthio)ethyl]piperazin-dihydrochlorid, Schmp. 230-233 °C.

**Beispiel 4:**

1-[4,4-Bis(4-fluorphenyl)butyl]-4-[2-(4-nitrophenylthio)ethyl]piperazin-dihydrochlorid, Schmp. 223-233 °C.

**Beispiel 5:**

1-[4,4-Bis(4-fluorphenyl)butyl]-4-[2-(3-chlorphenylthio)ethyl]piperazin-dihydrochlorid, Schmp. 239-249 °C.

## Ansprüche

1. Verbindung der Formel I,

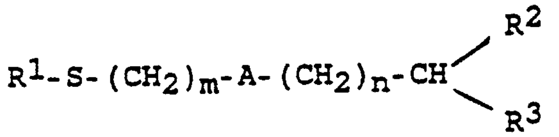

(I),

in welcher bedeuten:

$R^1$ ($C_1$-$C_6$)-Alkyl, geradkettig oder verzweigt, ($C_3$-$C_8$)-Cycloalkyl, ($C_2$-$C_6$)-Alkenyl, geradkettig oder verzweigt, ($C_5$-$C_8$)-Cycloalkenyl,

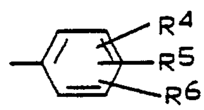

,

worin

$R^4$, $R^5$ und $R^6$ gleich oder verschieden unabhängig voneinander Wasserstoff, ($C_1$-$C_6$)-Alkyl, ($C_3$-$C_8$)-Cycloalkyl, Hydroxy, ($C_1$-$C_4$)-Alkoxy, ($C_1$-$C_4$)-Alkylthio, F, Cl, Br, J, Nitro, Cyano, Trifluormethyl, Formyl, Carboxyl, ($C_1$-$C_6$)-Alkoxycarbonyl, ($C_1$-$C_6$)-Acyl, Carbamoyl, N-Mono-oder N,N-Di-($C_1$-$C_6$)-Alkylcarbamoyl, Sulfo, ($C_1$-$C_6$)-Alkoxysulfonyl, Sulfamoyl, N-Mono-oder N,N-Di-($C_1$-$C_6$)-Alkylsulfamoyl, ($C_1$-$C_6$)-Alkylsulfinyl, ($C_1$-$C_6$)-Alkylsulfonyl, Amino, unsubstituiert oder substituiert mit ein oder zwei gleich-oder verschiedenartigen ($C_1$-$C_6$)-Alkyl-, ($C_1$-$C_6$)-Acyl-oder Aryl-, vorzugsweise Phenylgruppen bedeuten.

$R^2$ und $R^3$ gleich oder verschieden und unabhängig voneinander Phenyl, Phenyl-($C_1$-$C_4$)-alkyl, wobei der Phenylring jeweils unsubstituiert oder durch einen, zwei oder drei Substituenten aus der Gruppe ($C_1$-$C_4$)-Alkyl, ($C_1$-$C_4$)-Alkoxy, F, Cl, Br, J, Cyano, Nitro oder Trifluormethyl substituiert ist,

A ein Amin

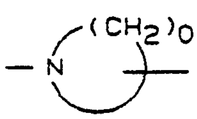

,

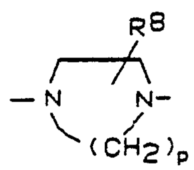

oder

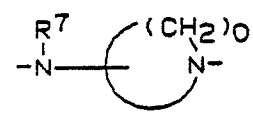

worin

$R^7$ Wasserstoff, ($C_1$-$C_6$)-Alkyl, Aryl, vorzugsweise Phenyl,

$R^8$ Wasserstoff, ($C_1$-$C_6$)-Alkyl, Formyl, ($C_1$-$C_6$)-Acyl, Carboxyl, ($C_1$-$C_6$)-Alkoxycarbonyl, Carbamoyl, N-Mono-oder N,N-Di-($C_1$-$C_6$)-Alkylcarbamoyl,

o 3, 4, 5, 6 oder 7,

p 2 oder 3 bedeuten,

m 2, 3 oder 4

n 1, 2, 3 oder 4,

sowie die Salze der Verbindungen der Formel I mit physiologisch verträglichen Säuren.

2. Verbindung der Formel I nach Anspruch 1, in welcher mindestens einer der Reste und Indices folgende Bedeutung hat:

$R^1$ ($C_3$-$C_8$)-Cycloalkyl,

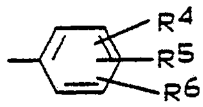

,

worin

$R^4$, $R^5$ und $R^6$ gleich oder verschieden unabhängig voneinander Wasserstoff, ($C_1$-$C_6$)-Alkyl, ($C_1$-$C_4$)-Alkoxy, ($C_1$-$C_4$)-Alkylthio, F, Cl, Br, J, Nitro, Cyano, Trifluormethyl, Formyl, Carboxyl, ($C_1$-$C_6$)-Alkoxycarbonyl, ($C_1$-$C_6$)-Acyl, Carbamoyl, N-Mono-oder N,N-Di-($C_1$-$C_6$)-Alkylcarbamoyl, Sulfo, Sulfamoyl, N-Mono-oder N,N-Di-($C_1$-$C_6$)-Alkylsulfamoyl, ($C_1$-$C_6$)-Alkylsulfinyl, ($C_1$-$C_6$)-Alkylsulfonyl bedeuten,

$R^2$ und $R^3$ gleich oder verschieden und unabhängig voneinander Phenyl, Phenyl-methyl, wobei der Phenylring jeweils unsubstituiert oder durch einen, zwei oder drei Substituenten aus der Gruppe Methyl, Ethyl, Methoxy, Ethoxy, F, Cl, Br, J, Cyano, Nitro oder Trifluormethyl substituiert ist,

A ein Amin

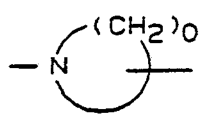 , 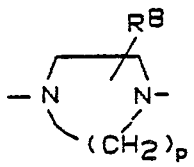 oder 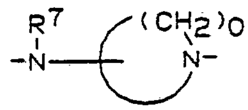

worin

$R^7$ Wasserstoff, Methyl, Ethyl,
$R^8$ Wasserstoff, Carboxyl, Carbamoyl,
o 4, 5 oder 6,
p 2 oder 3 bedeuten,
m 2, 3 oder 4
n 1, 2, 3 oder 4,

sowie die Salze der Verbindungen der Formel I mit physiologisch verträglichen Säuren.

3. Verbindung der Formel I nach Anspruch 1, in welcher mindestens einer der Substituenten und Indices folgende Bedeutung hat:

$R^1$ ($C_5$-$C_7$)-Cycloalkyl,

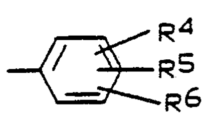

,

worin

$R^4$, $R^5$ und $R^6$ gleich oder verschieden unabhängig voneinander Wasserstoff, Methyl, Ethyl, Propyl, Isopropyl, N-Butyl, sec-Butyl, Isobutyl, tert. Butyl, Methoxy, Ethoxy, Methylthio, Fluor, Chlor, Brom, Jod, Nitro, Cyano, Trifluormethyl, Acetyl, Methylsulfonyl, Methylfulfinyl bedeuten,

$R^2$ und $R^3$ gleich oder verschieden und unabhängig voneinander Phenyl, Phenyl-methyl, wobei der Phenylring jeweils unsubstituiert oder durch einen, zwei oder drei Substituenten aus der Gruppe Methyl, Fluor, Chlor, Brom, Cyano, Nitro oder Trifluormethyl substituiert ist,

A ein Amin

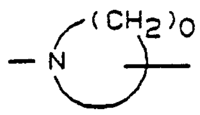 , 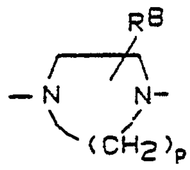 ,

worin

$R^8$ Wasserstoff, Carboxyl, Carbamoyl,
o 4, 5 oder 6,
p 2 oder 3 bedeuten,
m 2, 3 oder 4
n 2, 3 oder 4,

sowie die Salze der Verbindungen der Formel I mit physiologisch verträglichen Säuren.

4. Verbindung der Formel I nach Anspruch 1, in welcher mindestens einer der Substituenten oder Indices folgende Bedeutung hat:
$R^1$

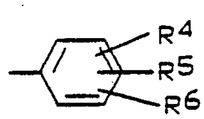 ,

worin

$R^4$, $R^5$ und $R^6$ gleich oder verschieden unabhängig voneinander Wasserstoff, Methyl, tert. Butyl, Methoxy, Methylthio, Fluor, Chlor, Brom, Nitro, Cyano, Trifluormethyl, Acetyl, Methylsulfonyl, Methylsulfinyl bedeuten,
$R^2$ und $R^3$ gleich oder verschieden unabhängig voneinander Phenyl, Phenyl-methyl, wobei der Phenylring jeweils unsubstituiert oder durch Fluor oder Trifluormethyl substituiert ist,
A ein Amin

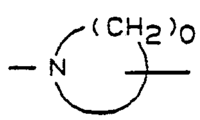 , 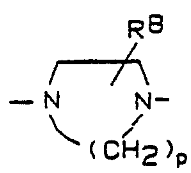 ,

worin

$R^8$ Wasserstoff,
o 5,
p 2 bedeuten,
m 2,
n 3,

sowie die Salze der Verbindungen der Formel I mit physiologisch verträglichen Säuren.

5. Verfahren zur Herstellung von Verbindungen der Formel I nach Anspruch 1, dadurch gekennzeichnet, daß man

a) eine Verbindung der Formel II,

$R^1 - S - (CH_2)_m - Y$ (II),

in welcher $R^1$ und m die gleiche Bedeutung wie in Formel I haben, und in welcher Y eine Abgangsgruppe, die nucleophil verdrängt werden kann, bedeutet, mit einer Verbindung der Formel III,

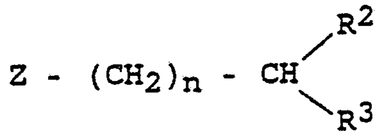

(III),

in welcher $R^2$, $R^3$ und n die gleiche Bedeutung wie in Formel I haben, und in welcher Z

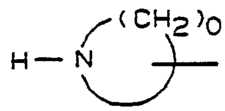 , 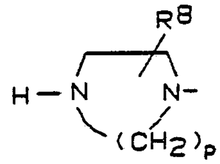 oder 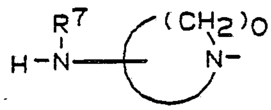

bedeutet, worin

$R^7$, $R^8$, o und p die gleiche Bedeutung wie in Formel I haben,
unter Bedingungen einer nucleophilen Substitution umsetzt, oder daß man

b) eine Verbindung der Formel IV,

$R^1 - S - (CH_2)_m - Z$ (IV)

in welcher $R^1$ und m die gleiche Bedeutung wie in Formel I und Z die gleiche Bedeutung wie in Formel III haben, mit einer Verbindung der Formel V,

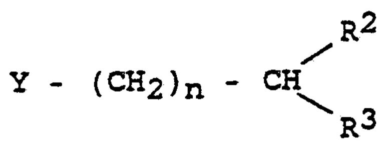

(V)

in welcher $R^2$, $R^3$ und n die gleiche Bedeutung wie in Formel I und Y die gleiche Bedeutung wie in Formel II haben,
unter den Bedingungen einer nucleophilen Substitution umsetzt,
oder daß man

c) eine Verbindung der Formel VI,

$R^1 - SH$ (VI)

in der $R^1$ die gleiche Bedeutung wie in Formel I besitzt,
mit einer Verbindung der Formel VII,

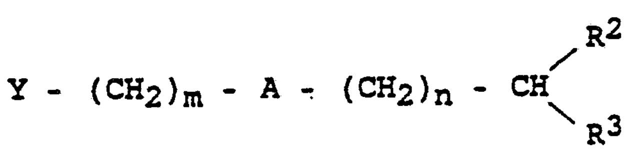

(VII)

in der $R^2$, $R^3$, A, m und n die gleiche Bedeutung wie in Formel I und Y die gleiche Bedeutung wie in Formel II haben, in Gegenwart einer schwachen bis mittelstarken Base umsetzt,
oder daß man

d) eine Verbindung der Formel VIII,

$R^1 - W$ (VIII)

in der $R^1$ die gleiche Bedeutung wie in Formel I besitzt und in der W eine Abgangsgruppe, die nucleophil verdrängt werden kann, bedeutet, mit einer Verbindung der Formel IX,

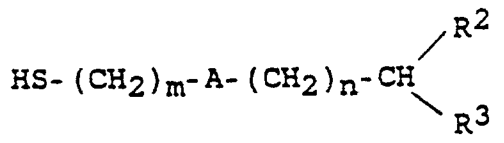

(IX),

in der $R^2$, $R^3$, A, m und n die gleiche Bedeutung wie in Formel I besitzen, unter Bedingungen einer nucleophilen Substitution umsetzt.

6. Heilmittel zur Behandlung von Herz-Kreislauf-und cerebrovaslulären Erkrankungen, gekennzeichnet durch einen wirksamen Gehalt einer Verbindung I nach Anspruch 1.

7. Verwendung einer Verbindung I nach Anspruch 1 zur Herstellung eines Medikaments zur Behandlung von Herz-Kreislauf-und cerebrovaskulären Erkrankungen.

Patentansprüche für folgende Vertragsstaaten : ES und GR:

1. Verfahren zur Herstellung von Verbindungen der Formel I

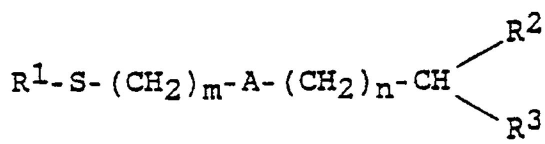

(I),

in welcher bedeuten:

$R^1$ $(C_1-C_6)$-Alkyl, geradkettig oder verzweigt, $(C_3-C_8)$-Cycloalkyl, $(C_2-C_6)$-Alkenyl, geradkettig oder verzweigt, $(C_5-C_8)$-Cycloalkenyl,

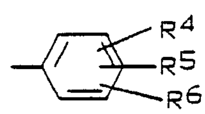

,

worin

$R^4$, $R^5$ und $R^6$ gleich oder verschieden unabhangig voneinander Wasserstoff, $(C_1-C_6)$-Alkyl, $(C_3-C_8)$-Cycloalkyl, Hydroxy, $(C_1-C_4)$-Alkoxy, $(C_1-C_4)$-Alkylthio, F, Cl, Br, J, Nitro, Cyano, Trifluormethyl, Formyl, Carboxyl, $(C_1-C_6)$-Alkoxycarbonyl, $(C_1-C_6)$-Acyl, Carbamoyl, N-Mono-oder N,N-Di-$(C_1-C_6)$-Alkylcarbamoyl, Sulfo, $(C_1-C_6)$-Alkoxysulfonyl, Sulfamoyl, N-Mono-oder N,N-Di-$(C_1-C_6)$-Alkylsulfamoyl, $(C_1-C_6)$-Alkylsulfinyl, $(C_1-C_6)$-Alkylsulfonyl, Amino, unsubstituiert oder substituiert mit ein oder zwei gleich-oder verschiedenartigen $(C_1-C_6)$-Alkyl-, $(C_1-C_6)$-Acyl-oder Aryl-, vorzugsweise Phenylgruppen bedeuten,

$R^2$ und $R^3$ gleich oder verschieden und unabhängig voneinander Phenyl, Phenyl-$(C_1-C_4)$-alkyl, wobei der Phenylring jeweils unsubstituiert oder durch einen, zwei oder drei Substituenten aus der Gruppe $(C_1-C_4)$-Alkyl, $(C_1-C_4)$-Alkoxy, F, Cl, Br, J, Cyano, Nitro oder Trifluormethyl substituiert ist,

A ein Amin

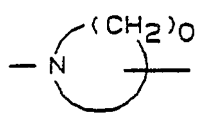 , 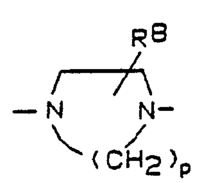 oder 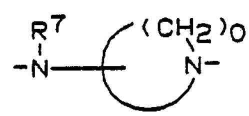

worin

$R^7$ Wasserstoff, $(C_1-C_6)$Alkyl, Aryl, vorzugsweise Phenyl,

$R^8$ Wasserstoff, $(C_1-C_6)$-Alkyl, Formyl, $(C_1-C_6)$-Acyl, Carboxyl, $(C_1-C_6)$-Alkoxycarbonyl, Carbamoyl, N-

Mono-oder N,N-Di-($C_1$-$C_6$)-Alkylcarbamoyl,
o 3, 4, 5, 6 oder 7,
p 2 oder 3 bedeuten,
m 2, 3 oder 4
n 1, 2, 3 oder 4,

sowie von Salzen der Verbindungen der Formel I mit physiologisch verträglichen Säuren, dadurch gekennzeichnet, daß man

a) eine Verbindung der Formel II,

$R^1$ - S - $(CH_2)_m$ -Y (II),

in welcher $R^1$ und m die gleiche Bedeutung wie in Formel I haben, und in welcher Y eine Abgangsgruppe, die nucleophil verdrängt werden kann, bedeutet, mit einer Verbindung der Formel III,

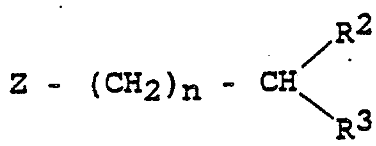 (III),

in welcher $R^2$, $R^3$ und n die gleiche Bedeutung wie in Formel I haben, und in welcher Z

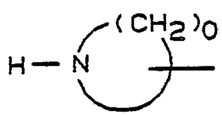 , 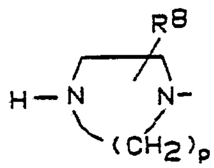 oder 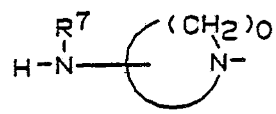

bedeutet, worin

$R^7$, $R^8$, o und p die gleiche Bedeutung wie in Formel I haben,
unter Bedingungen einer nucleophilen Substitution umsetzt, oder daß man

b) eine Verbindung der Formel IV,

$R^1$ - S - $(CH_2)_m$ -Z (IV)

in welcher $R^1$ und m die gleiche Bedeutung wie in Formel I und Z die gleiche Bedeutung wie in Formel III haben, mit einer Verbindung der Formel V,

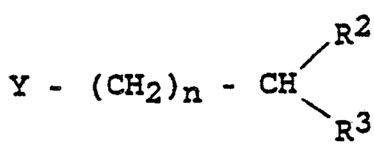 (V)

in welcher $R^2$, $R^3$ und n die gleiche Bedeutung wie in Formel I und Y die gleiche Bedeutung wie in Formel II haben,
unter den Bedingungen einer nucleophilen Substitution umsetzt,
oder daß man

c) eine Verbindung der Formel VI,

$R^1$ - SH (VI)

in der $R^1$ die gleiche Bedeutung wie in Formel I besitzt,
mit einer Verbindung der Formel VII,

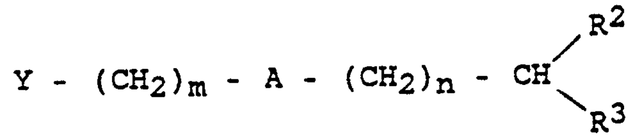 (VII)

in der $R^2$, $R^3$, A, m und n die gleiche Bedeutung wie in Formel I und Y die gleiche Bedeutung wie in Formel II haben, in Gegenwart einer schwachen bis mittelstarken Base umsetzt,

oder daß man

d) eine Verbindung der Formel VIII,

$R^1$ - W (VIII)

in der $R^1$ die gleiche Bedeutung wie in Formel I besitzt und in der W eine Abgangsgruppe, die nucleophil verdrängt werden kann, bedeutet, mit einer Verbindung der Formel IX,

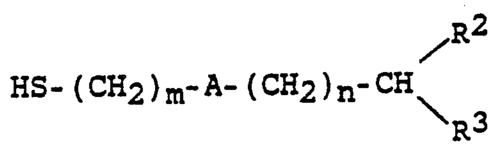 (IX),

in der $R^2$, $R^3$, A, m und n die gleiche Bedeutung wie in Formel I besitzen, unter Bedingungen einer nucleophilen Substitution umsetzt und gegebenenfalls in die Salze mit physiologisch verträglichen Säuren überführt.

2. Verfahren nach Anspruch 1, in welchem mindestens einer der Reste und Indices folgende Bedeutung hat:

$R^1$ ($C_3$-$C_8$)-Cycloalkyl,

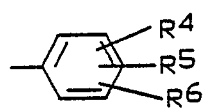 ,

worin

$R^4$, $R^5$ und $R^6$ gleich oder verschieden unabhängig voneinander Wasserstoff, ($C_1$-$C_6$)-Alkyl, ($C_1$-$C_4$)-Alkoxy, ($C_1$-$C_4$)-Alkylthio, F, Cl, Br, J, Nitro, Cyano, Trifluormethyl, Formyl, Carboxyl, ($C_1$-$C_6$)-Alkoxycarbonyl, ($C_1$-$C_6$)-Acyl, Carbamoyl, N-Mono-oder N,N-Di-($C_1$-$C_6$)-Alkylcarbamoyl, Sulfo, Sulfamoyl, N-Mono-oder N,N-Di-($C_1$-$C_6$)-Alkylsulfamoyl, ($C_1$-$C_6$)-Alkylsulfinyl, (Cl-$C_6$)-Alkylsulfonyl bedeuten,

$R^2$ und $R^3$ gleich oder verschieden und unabhängig voneinander Phenyl, Phenyl-methyl, wobei der Phenylring jeweils unsubstituiert oder durch einen, zwei oder drei Substituenten aus der Gruppe Methyl, Ethyl, Methoxy, Ethoxy, F, Cl, Br, J, Cyano, Nitro oder Trifluormethyl substituiert ist,

A ein Amin

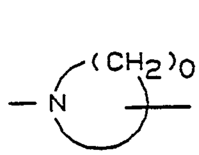 , 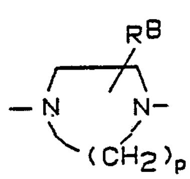 oder 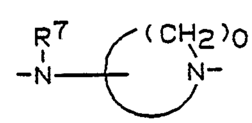

worin

$R^7$ Wasserstoff, Methyl, Ethyl,

$R^8$ Wasserstoff, Carboxyl, Carbamoyl,

o 4, 5 oder 6,

p 2 oder 3 bedeuten,
m  2, 3 oder 4
n  1, 2, 3 oder 4.

3. Verfahren nach Anspruch 1, in welchem mindestens einer der Substituenten und Indices folgende Bedeutung hat:
$R^1$ ($C_5$-$C_7$)-Cycloalkyl,

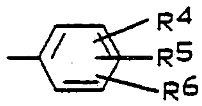

,

worin

$R^4$, $R^5$ und $R^6$ gleich oder verschieden unabhängig voneinander Wasserstoff, Methyl, Ethyl, Propyl, Isopropyl, N-Butyl, sec-Butyl, Isobutyl, tert.-Butyl, Methoxy, Ethoxy, Methylthio, Fluor, Chlor, Brom, Jod, Nitro, Cyano, Trifluormethyl, Acetyl, Methylsulfonyl, Methylsulfinyl bedeuten,

$R^2$ und $R^3$ gleich oder verschieden und unabhängig voneinander Phenyl, Phenyl-methyl, wobei der Phenylring jeweils unsubstituiert oder durch einen, zwei oder drei Substituenten aus der Gruppe Methyl, Fluor, Chlor, Brom, Cyano, Nitro oder Trifluormethyl substituiert ist,
A  ein Amin

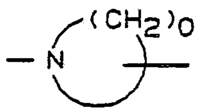

,

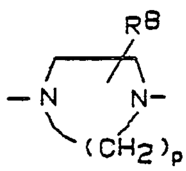

,

worin

$R^8$ Wasserstoff, Carboxyl, Carbamoyl,
o 4, 5 oder 6,
p 2 oder 3 bedeuten,
m  2, 3 oder 4
n  2, 3 oder 4.

4. Verfahren nach Anspruch 1, in welchem mindestens einer der Substituenten oder Indices folgende Bedeutung hat:
$R^1$

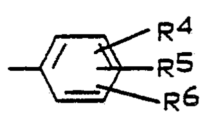

,

worin

$R^4$, $R^5$ und $R^6$ gleich oder verschieden unabhängig voneinander Wasserstoff, Methyl, tert. Butyl, Methoxy, Methylthio, Fluor, Chlor, Brom, Nitro, Cyano, Trifluormethyl, Acetyl, Methylsulfonyl, Methylsulfinyl bedeuten,

$R^2$ und $R^3$ gleich oder verschieden unabhängig voneinander Phenyl, Phenyl-methyl, wobei der Phenylring jeweils unsubstituiert oder durch Fluor oder Trifluormethyl substituiert ist,
A  ein Amin

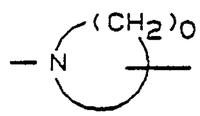 , 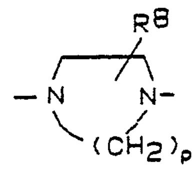 ,

worin

$R^8$ Wasserstoff,
o 5,
p 2 bedeuten,
m 2,
n 3.

5. Verfahren zum Herstellen eines Heilmittels zur Behandlung von Herz-Kreislauf-und cerebrovaskulären Erkrankungen, dadurch gekennzeichnet, daß man einen wirksamen Gehalt einer nach Anspruch 1 erhaltenen Verbindung I mit pharmazeutisch üblichen Zusatzstoffen mischt.

6. Verwendung einer Verbindung I nach Anspruch 1 zur Herstellung eines Medikaments zur Behandlung von Herz-Kreislauf-und cerebrovaskulären Erkrankungen.